# EUROPEAN PATENT SPECIFICATION

(11) **EP 0 638 124 B1**
(45) Date of publication and mention of the grant of the patent: **18.06.2003**
(21) Application number: 93910813.0
(22) Date of filing: 26.04.1993
(51) Int. Cl.: C12N 15/86, C12N 15/34, A01K 67/033, A01N 63/00, C12N 7/02

(54) **ORAL INFECTION OF INSECT LARVAE WITH PRE-OCCLUDED BACULOVIRUS PARTICLES**
MUNDINFEKTION DER INSEKTLARVEN MIT PRÄ-OKKLUDIERTEN BAKULOVIRUSPARTIKELN
INFECTION ORALE DE LARVES D'INSECTE PAR DES PARTICULES PRE-OCCLUSES DE BACULOVIRUS

(30) Priority: 29.04.1992 US 875691
(43) Date of publication of application: 15.02.1995
(73) Proprietor: BOYCE THOMPSON INSTITUTE FOR PLANT RESEARCH, INC., Ithaca, NY 14850 (US)
(72) Inventor: WOOD, H., Alan, Ithaca, NY 14850 (US)
(74) Representative: Jones, Graham H.
(86) International application number: US9303913
(87) International publication number: WO93022442

(56) References cited:
- WO-A-90/01556
- WO-A-90/14428
- WO-A-92/06181
- WO-A-92/15195
- WO-A-93/03144
- WO-A-93/09238
- NL-A- 8 800 198
- PHYTOPATHOLOGY vol. 82, no. 10, 21 August 1992, page 1175 VAN DAN HEUVEL , J.F.J.M. ET AL. 'Expression of discrete potyvirus gene products by a non-occluded recombinant baculovirus fed to Trichoplusia ni larvae'
- JOURNAL OF VIROLOGICAL METHODS vol. 42, no. 2-3, May 1993, AMSTERDAM NL pages 207 - 216 VAN DEN HEUVEL, J.F.J.M. ET AL. 'A simple and efficient procedure for the oral inoculation of Trichoplusia ni larvae with polyhedrin-negative recombinant baculovirus'
- HAMBLIN ET AL.: 'Co-Occlusion and Persistence of a Baculovirus Mutant Lacking the Polyhedrin Gene.' APPLIED AND ENVIRONMENTAL MICROBIOLOGY vol. 56, no. 10, 1990, pages 3057 - 3062
- BISHOP D.H.L ET AL.: 'Field-trials of genetically-engineered baculovirus insecticides.' THE RELEASE OF GENETICALLY-ENGINEERED MICROORGANISMS 1989, LONDON, pages 143 - 179

## Description

### Field of Invention

This invention relates to Pre-occluded baculovirus particles; particularly to Pre-occluded virus particles derived from baculoviruses which have been genetically altered such that they lack a functional polyhedrin or granulin gene, and to the use of said particles to infect insect larvae *per os*.

### Background of the Invention

It is generally recognized that there are two infectious forms of baculoviruses. Early in the replication cycle, baculoviruses produce nucleocapsids in the nucleus of a host cell which bud through the nuclear membrane into the cytoplasm and subsequently bud through the plasma membrane. These extracellular virus particles are referred to as budded virus or nonoccluded virus (NOV). The NOV are highly infectious to susceptible insect tissue culture cells. Insect larvae can be infected by injection of NOV into the hemocoel (circulatory system). However, NOV is generally recognized as non-infectious when administered *per os* (feeding) to larvae. Generally, only very low infections rates can be achieved with high concentrations of NOV using *per os* larval inoculations. Accordingly, the NOV have been used to infect larvae *per os* only in rare instances and in the absence of other alternatives.

Late in a baculovirus replication cycle, the nucleocapsids do not bud through the nuclear membrane, and they become membrane bound within the cell nucleus. At the same time baculovirus polyhedrin or granulin genes are transcribed and translated producing large amounts of protein which crystallize around the membrane-bound nucleocapsids present in the nucleus. These protein crystals containing virus particles have historically been referred to as granules with granulosis viruses and polyhedra with nuclear polyhedrosis viruses (both are types of baculoviruses). It is well known in the art that granulin and polyhedrin are functionally identical and are maintained in baculoviruses that are functional identical and have been termed differently due to historical precedent.

The granules and polyhedra of baculoviruses are used to efficiently infect susceptible host larvae through *per os* inoculations. The alkaline pH in the larval gut region results in dissolution of the crystalline protein surrounding the occluded virus (OV) particles. Following release from the crystal, the OV particles are able to efficiently infect larval gut cells. Although highly infectious *per os*, the OV particles are generally recognized as having an extremely low potential to initiate infections with tissue culture cells or by injection into the hemocoel of susceptible host larvae.

Accordingly, the baculovirus literature reflects the use of NOV as the form of inoculum to infect host larvae by hemocoelic injections or to infect insect tissue culture cells by addition to cell culture medium. And the polyhedra containing OV are used to infect susceptible host larvae by *per os* inoculations.

The construction of most baculovirus expression vector systems (BEV) has been based on replacement of the polyhedrin gene coding region with a foreign gene under the transcriptional control of the polyhedrin gene promoter (Pennock, G.D., Shoemaker, C., Miller, L.K. 1984, "Strong and regulated expression of *Escherichia coli* β-galactosidase in insect cells with a baculovirus vector", Mol. and Cell. Bio. 4:399-406; and Smith, G.E., Summers, M.D. and Fraser, M.J. 1983, "Production of human beta interferon in insect cells infected with a baculovirus expression vector", Molecular and Cell. Biol. 3:2156-2165). To date, hundreds of foreign genes have been expressed in BEV [Luckow, V.A. 1990, "Cloning and expression of heterologous genes in insect Cells with baculovirus vectors in Recombinant DNA Technology and Applications", (C. Ho, A. Prokop and R. Bajpai, eds.) McGraw-Hill, New York]. While the goal of most researchers has been to produce a protein for further study research or as a commercial product, there has been an increased interest in exploiting the BEV to express foreign proteins which would improve the pesticidal properties of the recombinant baculoviruses (Carbonell, L.F., Hodge, M.R., Tomalski, M.D., Miller, M.K., 1988, "Synthesis of a gene coding for an insect-specific scorpion neurotoxin and attempts to express it using baculovirus vectors", Gene 73:409-418; Hammock, B.D., Bonning, B.C., Possee, R.D., Hanzlik, T.N., Maeda, S., 1990, "Expression and effects of the juvenile hormone esterase in a baculovirus vector", Nature 344:458-461; Maeda, S., 1989, "Increased insecticidal effect by a recombinant baculovirus carrying a synthetic hormone gene", Biochem. Biophys. Res. Comm. 165:1177-1183; Maeda, S., Volrath, S.L., Hanzlik, T.N., Harper, S.A., Majima, K., Maddox, D.W., Hammock, B.D. and Fowler, E., 1991, "Insecticidal effects of an insect-specific neurotoxin expressed by a recombinant baculovirus", Virol. 184:777-780; Merryweather, A.T., Weyer, J., Harris, M.P.G., Hirst, M., Booth, T., Possee, R.D., 1990, "Construction of genetically engineered baculovirus insecticides containing the *Bacillus thuringiensis* ssp. *kurstaki* HD-73 delta endotoxin", J.Gen. Virol. 71:1534-1544; o'Reilly, D.R., Miller, L.K., 1989, "A Baculovirus blocks insect molting by producing ecdysteroid UDP-glucosyl transferase", Science 245:1110-1112; Stewart, L.M.D., Hirst, M., Ferber, M.L., Merryweather, A.T., Cayley, P.J., and Possee, R.D., 1991, "Construction of an improved baculovirus insecticide containing an insect-specific toxin gene", Nature 352:85-88; Tomalski, M.D. and Miller, L.K., 1991, "Insect paralysis by baculovirus-mediated expression of a mite neurotoxin gene", Nature 352:82-85; and O'Reilly, D.R. and Miller, L.K., 1991, "Improvement of a baculovirus pesticide by deletion of the EGT gene", Biotechnology 9:1086-1089). Baculoviruses are natural pathogens of many agriculturally important insect pests and are among the most promising alternatives to synthetic chemical pesticides (Wood, H.A. and R.R. Granados, 1991, "Genetically engineered baculoviruses as agents for pest control", Ann. Rev. Microbiol. 45:69-87).

In order to assess the pesticidal properties of BEV, insect host larvae must be inoculated at known virus dosages during a specified period of time and the time course of infection followed. Deletion of the polyhedrin gene from BEV and the resultant lack of polyhedra production (and, therefore, the resultant absence of OVs) has been problematic to performing larval bioassays because the naturally occurring NOV is not very infections *per os.* Additionally, it has been proposed to infect insect larvae with BEV to produce proteins of commercial interest (Maeda, S., Kawai, T., Obinata, M., Fujiwara, H., Horiuchi, T., Seki, Y., Sato, Y., and Furusawa, M., 1985, "Production of human alpha-interferon in silkworm using a baculovirus vector", Nature 315:592-594). Currently as demonstrated in all the references listed above, the common method of infecting larvae with BEV containing pesticidal or other foreign genes but lacking a functional polyhedrin gene (polyhedrin-minus virus) is by hemocoelic injection of larvae with the NOV. Alternatively, diet contamination with NOV has been used to establish infections, but very large doses are required due to the poor infectivity of this form of the virus by the oral route [Granados, R.R. and Williams, K.A., 1986, *"In vivo* infection and replication of baculoviruses" in The Biology of Baculoviruses Vol. I, Biological Properties and Molecular Biology" (R.R. Granados and B.A. Federici, eds.), CRC Press, Boca Raton, Florida].

The emphasis in the art has been on meeting the need for a method to simply and efficiently infect host insect larvae with polyhedrin-minus BEV isolates using *per os* inoculation procedures. The development of such a method would provide an efficient and simple means to: a) evaluate the pesticidal properties of a polyhedrin-minus BEV expressing foreign pesticidal gene products, b) infect large numbers of host insect larvae with polyhedrin-minus BEV expressing foreign proteins of commercial value (thereby, providing an alternative to BEV protein production in insect tissue culture cells), and c) infect insect larvae with polyhedrin-minus BEV expressing foreign pesticidal gene products under field conditions.

The production of commercial protein products in insect larvae following infection with a polyhedrin-minus BEV currently would require larval NOV injections, a costly and laborious process. The commercial pesticide application of a polyhedrin-minus BEV expressing pesticidal proteins has been considered not possible by those schooled in the art. Such a pesticidal product would be ideal in that the progeny NOV (no polyhedra formed) would not persist in the environment [Bishop, D.H.L., Entwistle, P.F., Cameron, I.R., Allen, C.J., Possee, R.D., 1988, "Field trials of genetically-engineered baculovirus insecticides", in The Release of Genetically-engineered Micro-organisms, (eds. M. Sussman, C.H. Collins, F.A. Skinner, D.E. Stewart-Tull), Academic Press, NY; Hamblin, M., van Beek, N.A.M., Hughes, P.R., and Wood, H.A., 1990 "Co-occlusion and persistence of a baculovirus mutant lacking the polyhedrin gene", Applied and Environ. Microbiol. 56:3057-3062]. Because of the teachings of the current art, skilled artisans have directed much of their energies at either co-occlusion of polyhedrin-minus BEV with wild type virions (see above Hamblin *et al*. 1990, Miller, U.S. Patent No. 5,071,748, Issued 12-10-91, Filed May 15, 1989, and ACS and Price, International Application Published Under The Patent Cooperation Treaty, International Publication Number WO 90/01556, International Publication Date February 22, 1990, International Application Number PCT/US89/03542) or constructing BEV which express foreign genes and a polyhedrin gene (polyhedrin-plus). The use of a polyhedrin-plus BEV as a commercial pesticide raises serious questions regarding persistence and competition of an engineered virus in the environment, from both ecological and health-safety standpoints.

It has been known in the art that late in the replication cycle of occluded baculoviruses, nucleocapsids become enveloped in bundles within the nucleus prior to being occluded by polyhedrin or granulin protein [Federici, B.A. 1986, Ultrastructure of baculoviruses in "The Biology of Baculoviruses Vol. I, Biological Properties and Molecular Biology" (R.R. Granados and B.A. Federici, eds.), CRC Press, Boca Raton, Florida]. These virions are equally infectious *per os* when fed in polyhedra or following alkaline release and purification from the polyhedrin protein (Van Beek, N.A.M., Derksen, A.C.G., Granados, R.R. and Hughes, P.R., 1987, "Alkaline liberated baculoviruses particles retained their infectivity *per os* for neonate lepidopterous larvae", J. Invertebr. Pathol. 50:339-340). However, prior to this invention it was not known that these membrane bound virus particles destined to be occluded [which we refer to as pre-occluded virus (POV) particles], but not occluded if it derives from a polyhedrin-minus virus, were infectious *per os.* Furthermore, it was assumed in the art that infectious POV did not develop during replication of a polyhedrin-minus baculovirus; the continual and exhausting efforts in the art directed towards constructing pesticidal BEV with a polyhedrin gene in order to evaluate the biological properties as well as performing infectivity assays of polyhedrin-minus BEV by NOV injections or diet surface contamination with high concentrations of NOV illustrates the assumption of the non-existence of a POV during replication of a polyhedrin-minus baculovirus.

However, recently Hamblin et al. (Hamblin et al., 1990, Applied and Environmental Microbiology, vol. 56(10), p. 3057-3062) disclosed co-occlusion and persistence of a baculovirus mutant lacking the polyhedrin gene. Furthermore, are field trials using polyhedrin - minus baculo = viruses revienved in "The release of genetically - engineered microorganisms", M. Sussman et al. (Eds.), Academic Press (London), 1988, article by Bishop et al., p. 143-179.

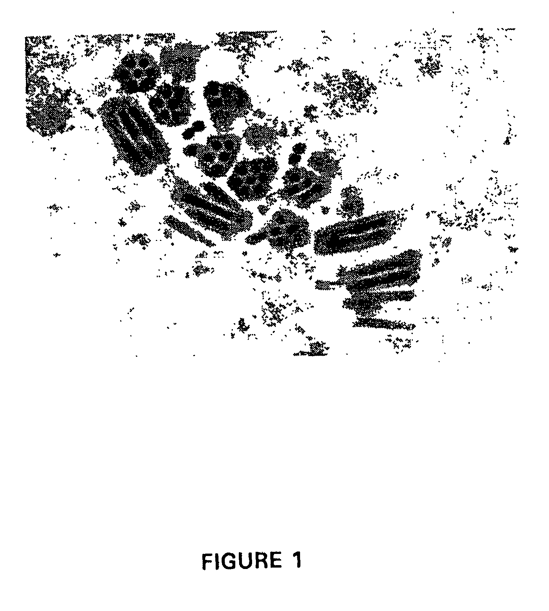

The inventor of the subject invention shows in the subject application that the formation and accumulation of POV occurs during the replication of a polyhedrin-minus baculoviruses (Fig.1), and that these POV can be stabilised by freeze-drying and are infectious *per os* using the neonate droplet feeding method (Hughes, P.R., Wood, H.A., 1981, "A synchronous peroral technique for the bioassay insect viruses", J. Invert. Pathol. 37:154-159; and Hughes, P.R., Van Beek, N.A.M., Wood, H.A., 1986, "A modified droplet feeding method for rapid assay of *Bacillus thuringiensis* and baculoviruses in noctuid larvae", J. Invert. Pathol. 48:187-192). Thus, this invention represents a simple solution to a problem in a manner which is contrary to the teachings and expectations of the prior art.

### Summary of the Invention

A method of infecting insects is disclosed; the method utilizes a form of a baculovirus which is highly efficient at establishing infection and is normally destined to become occluded within the polyhedrin or granulin -- Pre-occluded virus particles (POV). Specifically, the POV produced during replication of polyhedrin-minus (genetic alteration resulting in nonfunctional polyhedrin or granulin gene) baculovirus is highly infectious to insect larvae when administered *per os*. The discovery of a POV form of polyhedrin-minus baculoviruses was essential to our invention of the novel method of infecting insect larvae *per os* using the POV form of polyhedrin-minus baculovirus, and as such is also described and taught in the specifications.

It is therefore an object of this invention to disclose a novel method of infecting insects *per os* with POV formed during the replication of polyhedrin-minus or granulin-minus baculoviruses.

It is another object of this invention to disclose a novel method of making POV derived from polyhedrin-minus or granulin-minus baculovirus during the course of replication in insect tissue culture cells.

It is another object of this invention to disclose a novel method of making POV derived from polyhedrin-minus or granulin-minus baculovirus during the course of replication in insect larvae.

It is yet another object of this invention to disclose a novel method of making POV derived from polyhedrin-minus or granulin-minus baculovirus during the course of replication in insect pupae.

It is yet another object of this invention to disclose a novel pesticide; wherein said POV derived from a polyhedrin-minus or granulin-minus baculovirus is the active agent of said pesticide.

Further objects of the invention will be set forth in the description which follows, and will become apparent to those skilled in the art upon examination of the specification or by practice of the invention.

### Brief Description of the Drawings

Figure 1. An electron micrograph of Sf-21 insect tissue culture cells at 30 hours post infection with a recombinant baculovirus (polyhedrin-minus Ac-E10 isolates of *Autographa californica* nuclear polyhedrosis virus) which lacks a functional polyhedrin gene; the cells contain large numbers of pre-occluded virus particles.

### Detailed Description of the Invention

The naturally occurring, polyhedrin positive virus (polyhedrin-plus) was the 1A clone of AcNPV (Wood, H.A., 1980, "Isolation and replication of an occlusion body-deficient mutant of the *Autographa californica* nuclear polyhedrosis virus", Virol. 105-338-344). The polyhedrin-minus strain (Ac-E10) is a deletion mutant of the 1A clone which lacks a functional polyhedrin gene (Hamblin, M., van Beek, N.A.M., Hughes, P.R., Wood, H.A., 1990, "Co-occlusion and persistence of a baculovirus mutant lacking the polyhedrin gene", Appl. and Environ. Microbio. 56:3057-3062). The β-galactosidase producing, polyhedrin-minus virus (E2-β-gal) was obtained from MicroGenesys (Meriden, CT). The E2-β-gal virus lacks a functional polyhedrin gene and expresses β-galactosidase under the transcriptional control of the polyhedrin gene promoter. *Spodoptera frugiperda* cells, IPLB-SF-21 (Sf-21) (Vaughn, J.L., Goodwin, R.H., Tompkins, G.J., McCawley, P., 1977, "The establishment of two cell lines from the insect *Spodoptera frugiperda (Lepidoptera:Noctuidae)",* In vitro 13: 213-217), were cultured in modified TNMFH medium (Wood, H.A., 1980, "Isolation and replication of an occlusion body-deficient mutant of the *Autographa californica* nuclear polyhedrosis virus", Virol. 105-338-344). Tissue culture plaque assays to determine NOV (nonoccluded or budded virus) titers were performed according to Wood (Wood, H.A., 1977, "An agar overlay plaque assay method for *Autographa californica* nuclear polyhedrosis virus", J. Invert. Pathol. 29:304-307).

The POV were prepared by infecting 9 x 10⁶ Sf-21 cells with the Ac-E10 (polyhedrin-minus) virus in 6-well multiwell plates (1.5x10⁶ cells/well) at a multiplicity of infection of 10 plaque forming units (pfu) per cell and incubating the infected cells for 30 hours. This time period post infection was selected because it is known that polyhedrin protein expression occurs prior to 16 hours post-infection, and occlusion of OV particles takes place prior to 24 hours post infection (page 305 of Wood, H.A., 1977, "An agar overlay plaque assay method for *Autographa californica* nuclear polyhedrosis virus", J. Invert. Pathol. 29:304-307). Given the knowledge that POV particles are formed in polyhedrin-minus baculovirus infected cells, it was therefore expected that large numbers of POV particles would accumulate in the nucleus by 30 hours post infection. Higher concentrations of POV per cell may be reached at later time periods post infection.

Figure 1 is an electron micrograph of Sf-21 insect tissue culture cells at 30 hours post infection with a recombinant baculovirus (polyhedrin-minus Ac-E10 isolate of *Autographa californica* nuclear polyhedrosis virus) which lacks a functional polyhedrin gene; the Ac-E10 infected cells contain large numbers of pre-occluded virus particles within their nucleus. The electron micrograph clearly shows the accumulation of multiple nucleocapsids enveloped within a single membrane. These particles are identical in appearance to those found in the cell nucleus of cells infected with a polyhedrin-plus virus (wild type virus) (page 78, Figure 6A and 6E in Federici, B.A. 1986, Ultrastructure of baculoviruses in "The Biology of Baculoviruses Vol. I, Biological Properties and Molecular Biology" [R.R. Granados and B.A. Federici, eds.], CRC Press, Boca Raton, Florida). Furthermore, it is known in the art that baculovirus NOV particles are constructed of single nucleocapsids bound within a membrane (page 78, Figure 6F in Federici, B.A. 1986 Ultrastructure of baculoviruses in "The Biology of Baculoviruses Vol. I, Biological Properties and Molecular Biology" [R. R. Granados and B. A. Federici, eds.], CRC Press, Boca Raton, Fla.) and not multiple nucleocapsids bound within a membrane. Therefore, the multiple nucleocapsids (the black circles and rods [circles are views along the axis of the nucleocapsids and rods are longitudinal views of the nucleocapsids]) bound within a membrane (the dark gray surrounding each bundle of black circles or rods) in Figure 1 are clearly POV particles derived from a polyhedrin-minus baculovirus (Ac-E10).

The infected cell samples containing the POV were harvested by twice pelleting the cells and resuspending the samples in tissue culture medium. Following harvesting, the cell samples were subjected to sonication sufficient to disrupt them without altering the infectivity of the POV. The samples were then serially diluted and fed *per os* to *Trichoplusia ni* (cabbage looper) neonate larvae in a neonate feeding assay (Hughes, P.R., Wood, H.A., 1981, "A synchronous peroral technique for the bioassay insect viruses", J. Invert. Pathol. 37:154-159; and Hughes, P.R., Van Beek, N.A.M., Wood, H.A., 1986, "A modified droplet feeding method for rapid assay of *Bacillus thuringiensis* and baculoviruses in noctuid larvae", J. Invert. Pathol. 48:187-192). The volume ingested by the neonate larvae was determined (Van Beek, N.A.M., and Hughes, P.R., 1986, "Determination of the volume ingested by neonate lepidopterous larvae using the fluorescent dye sodium fluorescein", J. Invert. Pathol. 48:249-251). Based on the volume ingested per larvae, it was determined that following ingestion of approximately 1 cell equivalent 93% (28/30) of the larvae became infected.

Since the Ac-E10 POV preparation also contained NOV, tissue culture plaque assays were performed to determine the number of plaque forming units of NOV ingested. The volume of the inoculum which infected 93% of the larvae was determined to contain between 3.5-5.6 plaque forming units of Ac-E10 NOV. When equivalent titers of NOV samples (prepared such that no POV were in the samples) or lysates from mock infected cells were used to inoculate test larvae, no deaths occurred. Accordingly, the high titer of infectious virus in the lysate of Ac-E10 infected cells was the result of the POV and not the result of NOV. This is consistent with the known art that NOV are not very infectious *per os.* For example, 3 X 10⁴⁻⁶ plaque forming units of NOV were used to infect *Trichoplusia ni* larvae *per os* (page 460, Figure 4 in Hammock, B.D., Bonning, B.C., Possee, R.D., Hanzlik, T.N., Maeda, S., 19990, "Expression and effects of the juvenile hormone esterase in a baculovirus vector", Nature 344:458-461).

The method of infecting insect larvae *per os* with a form of a baculovirus which is highly efficient at establishing infection and is normally destined to become occluded within the polyhedrin or granulin, therefore, comprises the steps of: a. infecting an insect host, wherein said insect host are selected from a group consisting of insect larvae, insect pupae, and *in vitro* insect cells, with a baculoviruses lacking a functional polyhedrin or granulin gene (polyhedrin-minus baculoviruses [e.g. NOV and polyhedrin-minus POV]), b. waiting for the progeny Pre-occluded virus particles derived from said baculovirus to form in said insect host, c. disrupting said insect host cells, which are housing said Pre-occluded virus particles, and d. feeding said disrupted cells and Pre-occluded virus particles *per os* to insect larvae.

Tissue culture POV samples diluted in tissue culture medium were held at 4 degrees centigrade for 2-3 weeks and reassayed to determine the stability of the infectivity of POV. The infectivity of the POV samples remained unaltered. Following cell harvesting, washing and sonication of Ac-E10 (polyhedrin-minus AcNPV) infected tissue culture cells, the samples were processed by freeze drying. Following rehydration, the POV sample remained infectious based on neonate feeding bioassays (infection and death of 16 of 24 test larvae resulted); therefore, the infectivity of POV can be stabilized by processing such as freeze-drying. Processing may be before or after disruption of the cells.

Infectious POV can also be obtained from homogenates of larvae or pupae infected with Ac-E10 or other polyhedrin-minus BEV, however, following death of the larvae or pupae, the infectivity is lost unless the preparation is diluted. The loss of infectivity presumably is caused by protease activity in the insect homogenate.

Following infection of insect larvae *per os* with POV from a polyhedrin-minus baculovirus, or infection of tissue culture cells with NOV from a polyhedrin-minus baculovirus or insect pupae with NOV from a polyhedrin-minus baculovirus, progeny NOV and POV particles are formed. If the infecting baculovirus contains an expressed foreign gene insert (BEV), in addition to NOV and POV, the foreign gene product is also produced. For example, when POV of polyhedrin-minus baculovirus maintaining a foreign gene infects insect larvae *per os* (or when NOV maintaining an insert infects insect pupae or insect tissue culture cells) the foreign protein is expressed. When the POV of a polyhedrin-minus baculovirus containing a gene coding for β-galactosidase (E2-β-gal recombinant virus) was fed to insect larvae (*Trichopulsia ni*) *per os*, β-galactosidase was produced (the POV was obtained and infected the insect larvae using the method of infecting insect larvae *per os* as taught above in the specifications). This expression of the inserted gene of baculoviruses during replication of baculoviruses in an insect host is well known in the art [Luckow, V.A. 1990, "Cloning and expression of heterologous genes in insect Cells with baculovirus vectors in Recombinant DNA Technology and Applications", (C. Ho, A. Prokop and R. Bajpai, eds.) McGraw-Hill, New York, pages 1-25]. The expressed gene products include therapeutic/vaccine proteins such as human immunodeficient virus (HIV, HTLV-III) envelope protein, influenza virus hemagglutin protein, canine parvovirus coat protein, α- and β-interferon and interleukin-2 as well as pesticidal gene products such as Belt insectotoxin 1, juvenile hormone esterase, *Androctonus australis* insect neurotoxin, and *Pyemotes tritici* insect neurotoxin.

The data clearly demonstrate the utility of using POV of polyhedrin-minus, recombinant baculoviruses as pesticides and for the production of gene products such as those just mentioned above. In commercial practice a gene coding for a product, which would increase the pesticidal action of the virus or be a desired protein product, could be inserted into a polyhedrin-minus baculovirus which would produce POV. Following the teachings of this invention polyhedrin-minus POV may then be used to inoculate *per os* large numbers of insect larvae simply and cost effectively and express the inserted gene. Furthermore, polyhedrin-minus POV is also safe environmentally, because such a POV will not be occluded, and therefore it will not persist in the environment after it has infected and killed its host. Therefore, in addition to the efficient use of POV from polyhedrin-minus baculoviruses in infecting insect larvae *per os* for expressing a gene insert, the POV also has the additional advantage of being an environmentally safe genetically engineered baculovirus pesticide.

## Claims

1. A method of infecting an insect larva *per os* with at least one pre-occluded virus particle, a form of a baculovirus which is highly efficient at establishing infection and is normally destined to become occluded within the polyhedrin, comprising the steps of:
a. infecting at least one insect larva with a baculovirus lacking a functional polyhedrin or granulin gene;
b. incubating the infected insect larva to allow for the pre-occluded virus particles derived from said baculovirus to form in said insect larva;
c. disrupting said infected insect larva, which is housing said pre-occluded virus particles;
d. stabilizing the infectivity of the pre-occluded virus particles in the disrupted larva by freeze-drying; and
e. feeding the disrupted larval cells and said stabilized pre-occluded virus particles *per os* an insect larva.

2. The method of claim 1, wherein, in step (b), the infected insect larva is incubated for at least thirty hours.

3. The method of claim 1 wherein said pre-occluded virus particle maintains and expresses a foreign gene product but not a polyhedrin or granulin gene.

4. The method of claim 3, wherein said foreign gene codes for a pesticidal protein.

5. The method of claim 3, wherein said foreign gene codes for an enzyme or therapeutic protein.

6. The method of claim 3, wherein said foreign gene codes for a protein that may be used as a vaccine.

7. A method of making at least one stabilized pre-occluded virus particle, a form of a baculovirus which is highly efficient at establishing infection and is normally destined to become occluded within the polyhedrin, comprising the steps of:
a. infecting at least one insect larva with baculoviruses lacking a functional polyhedrin or granulin gene;
b. incubating the infected insect larva to allow for the pre-occluded virus particles derived from said baculovirus to form in said insect larva;
c. disrupting said insect host larva, which is housing said pre-occluded virus particles; and
d. stabilizing the infectivity of the pre-occluded virus particles in the disrupted larva by freeze-drying.

8. The method of claim 7, wherein, in step (b), the infected insect larva is incubated for at least thirty hours.

9. A pesticide comprising disrupted insect cells maintaining stabilized pre-occluded virus particles, a form of a baculovirus which is highly efficient at establishing infection and is normally destined to become occluded within the polyhedrin, wherein said insect cells have been infected with a polyhedrin-minus or granulin-minus baculovirus and freeze-dried to stabilize the pre-occluded virus particles.

10. The pesticide of claim 9, wherein said pre-occluded virus particles are unable to persist in the environment.

11. A method of pest control which comprises applying a stabilized freeze-dried pre-occluded baculovirus lacking a functional polyhedrin gene.

12. The method of claim 11, wherein said pre-occluded baculovirus is produced by the method of claim 7.

13. The method of claim 11, wherein said pre-occluded baculovirus is unable to persist in the environment.

## Patentansprüche

1. Verfahren zum Infizieren einer Insektenlarve *per os* mit mindestens einem prä-okkludierten Virus-Partikel, einer Form eines Baculovirus, der hochgradig effizient ist, eine Infektion herbeizuführen, und der normalerweise dazu bestimmt ist, innerhalb des Polyhedrins okkludiert zu werden, umfassend die folgenden Schritte:
a. Infizieren mindestens einer Insektenlarve mit einem Baculovirus, dem ein funktionelles Polyhedrin- oder Granulin-Gen fehlt.
b. Inkubieren der infizierten Insektenlarve, um die Bildung der prä-okkludierten Virus-Partikel, die sich von dem besagten Baculovirus ableiten, in der besagten Insektenlarve zu ermöglichen;
c. Aufschließen der besagten infizierten Insektenlarve, welche die besagten prä-okkludierten Virus-Partikel beherbergt;
d. Stabilisieren der Infektivität der prä-okkludierten Virus-Partikel in der aufgeschlossenen Larve durch Gefriertrocknung; und
e. Verfüttern der aufgeschlossenen Larvenzellen und des besagten stabilisierten prä-okkludierten Virus-Partikels *per os* an die Insektenlarve.

2. Verfahren nach Anspruch 1, wobei in Schritt (b) die infizierte Insektenlarve für mindestens 30 Stunden inkubiert wird.

3. Verfahren nach Anspruch 1, wobei die besagten prä-okkludierten Virus-Partikel ein fremdes Gen-Produkt beibehalten und exprimieren, jedoch kein Polyhedrin- oder Granulin-Gen.

4. Verfahren nach Anspruch 3, wobei das besagte fremde Gen für ein pestizides Protein codiert.

5. Verfahren nach Anspruch 3, wobei das besagte fremde Gen für ein Enzym oder ein therapeutisches Protein codiert.

6. Verfahren nach Anspruch 3, wobei das besagte fremde Gen für ein Protein codiert, das als Impfstoff verwendet werden kann.

7. Verfahren zur Herstellung von mindestens einem stabilisierten prä-okkludierten Virus-Partikel, einer Form eines Baculovirus, der hochgradig wirksam ist, um Infektionen herbeizuführen, und der normalerweise dazu bestimmt ist, innerhalb des Polyhedrins okkludiert zu werden, folgende Schritte umfassend:
a. Infizieren mindestens einer Insektenlarve mit Baculoviren, denen ein funktionelles Polyhedrinoder Granulin-Gen fehlt;
b. Inkubieren der infizierten Insektenlarve, um die Bildung der prä-okkludierten Virus-Partikel, die sich von dem besagten Baculovirus ableiten, in der besagten Insektenlarve zu ermöglichen;
c. Aufschließen dieser Insekten-Wirtslarve, welche die besagten prä-okkludierten Virus-Partikel beherbergt; und
d. Stabilisieren der Infektivität der prä-okkludierten Virus-Partikel in der aufgeschlossenen Larve durch Gefriertrocknung.

8. Verfahren nach Anspruch 7, wobei in Schritt (b) die infizierte Insektenlarve für mindestens 30 Stunden inkubiert wird.

9. Pestizid, das aufgeschlossene Insektenzellen umfaßt, welche stabilisierte, prä-okkludierte Virus-Partikel beibehalten, eine Form eines Baculovirus, der hochgradig wirksam ist, um eine Infektion herbeizuführen, und der normalerweise dazu bestimmt ist, innerhalb des Polyhedrins okkludiert zu werden, wobei die besagten Insektenzellen mit einem Polyhedrin-Minus oder Granulin-Minus Baculovirus infiziert worden sind, und gefriergetrocknet worden sind, um die prä-okkludierten Virus-Partikel zu stabilisieren.

10. Pestizid nach Anspruch 9, wobei die besagten prä-okkludierten Virus-Partikel unfähig sind, in der Umwelt zu persistieren.

11. Verfahren zur Schädlingskontrolle, das die Anwendung eines stabilisierten prä-okkludierten Baculovirus umfaßt, dem ein funktionelles Polyhedrin-Gen fehlt.

12. Verfahren nach Anspruch 11, wobei das besagte prä-okkludierte Baculovirus durch das Verfahren nach Anspruch 7 hergestellt wird.

13. Verfahren nach Anspruch 11, wobei der besagte prä-okkludierte Baculovirus unfähig ist, in der Umwelt zu persistieren.

## Revendications

1. Procédé d'infection d'une larve d'insecte *per os* avec au moins une particule de virus pré-occluse, une forme d'un baculovirus qui est hautement efficace à établir une infection et qui est normalement destiné à devenir occlus à l'intérieur d'une polyhédrine, comprenant les étapes :
a. d'infection d'au moins une larve d'insecte par un baculovirus auquel un gène fonctionnel de polyhédrine ou de granuline fait défaut,
b. d'incubation de la larve d'insecte infectée pour permettre aux particules de virus pré-occluses dérivées dudit baculovirus de se former dans la larve d'insecte,
c. de rupture de ladite larve d'insecte infectée qui est l'hôte desdites particules de virus pré-occluses,
d. de stabilisation de l'infectivité des particules de virus pré-occluses dans la larve rompue, par lyophilisation, et
e. d'alimentation des cellules de larve rompues et des particules de virus pré-occluses stabilisées *per os* à une larve d'insecte.

2. Procédé suivant la revendication 1, dans lequel, dans l'étape (b), la larve d'insecte infectée est incubée pendant au moins trente heures.

3. Procédé suivant la revendication 1, dans lequel la particule de virus pré-occluse maintient et exprime un produit de gène étranger, mais pas un gène de polyhédrine ou de granuline.

4. Procédé suivant la revendication 3, dans lequel ledit gène étranger code pour une protéine pesticide.

5. Procédé suivant la revendication 3, dans lequel le gène étranger code pour une enzyme ou protéine thérapeutique.

6. Procédé suivant la revendication 3, dans lequel le gène étranger code pour une protéine qui peut être utilisée comme un vaccin.

7. Procédé de réalisation d'au moins une particule de virus pré-occluse stabilisée, une forme d'un baculovirus qui est hautement efficace à établir une infection et est normalement destiné à devenir occlus à l'intérieur de la polyhédrine, comprenant les étapes :
a. d'infection d'au moins une larve d'insecte par des baculovirus auxquels un gène fonctionnel de polyhédrine ou de granuline fait défaut,
b. d'incubation de la larve d'insecte infectée pour permettre aux particules de virus pré-occluses dérivées du baculovirus de se former dans la larve d'insecte,
c. de rupture de la larve d'insecte hôte, qui est l'hôte desdites particules de virus pré-occluses, et
d. de stabilisation de l'infectivité des particules de virus pré-occluses dans la larve rompue, par lyophilisation.

8. Procédé suivant la revendication 7, dans lequel, dans l'étape (b), la larve d'insecte infectée est incubée pendant au moins 30 heures.

9. Pesticide comprenant des cellules d'insecte rompues qui maintiennent des particules de virus pré-occluses stabilisées, une forme d'un baculovirus qui est hautement efficace à établir une infection et est normalement destiné à devenir occlus à l'intérieur de la polyhédrine, dans lequel les cellules d'insecte ont été infectées par un baculovirus polyhédrine-négatif ou granuline-négatif et lyophilisées pour stabiliser les particules de virus pré-occluses.

10. Pesticide suivant la revendication 9, dans lequel les particules de virus pré-occluses sont incapables de persister dans l'environnement.

11. Procédé de désinsectisation qui comprend une application d'un baculovirus pré-occlus stabilisé, auquel un gène fonctionnel de polyhédrine fait défaut.

12. Procédé suivant la revendication 11, dans lequel le baculovirus pré-occlus est produit par le procédé suivant la revendication 7.

13. Procédé suivant la revendication 11, dans lequel le baculovirus pré-occlus est incapable de persister dans l'environnement.
